# EUROPÄISCHE PATENTANMELDUNG

(11) **EP 2 251 669 A2**
(43) Veröffentlichungstag der Anmeldung: **17.11.2010**
(21) Anmeldenummer: 10001973.6
(22) Anmeldetag: 26.02.2010
(51) Int. Cl.: G01N 9/24

(54) **Vorrihtung zum Bestimmen der Rohdichte des Materials in einem Plattenstrand**

(30) Priorität: 13.05.2009 DE 202009006911 U
(71) Anmelder: FAGUS-GRECON GRETEN GMBH & CO. KG, 31061 Alfeld (DE)
(72) Erfinder: Bergmann, Bernd, 30900 Wedemark (DE); Greten, Ernst, 31061 Alfeld (DE)
(74) Vertreter: Jabbusch, Matthias

(57) **Zusammenfassung**

Bei einer Vorrichtung zum Bestimmen der Rohdichte des Materials in einem Plattenstrang, umfassend eine Einrichtung zur Messung der Plattendicke des Materials ist vorgesehen, dass sie zumindest eine Röntgenquelle aufweist, dass die Röntgenquelle eine fächerförmige Strahlung aussendet und dass auf der in Bezug auf die Röntgenquelle anderen Seite des Plattenstranges zumindest ein Detektor angeordnet ist.

Mit dieser Vorrichtung ist die Rohdichte in kurzer Zeit bestimmbar.

## Beschreibung

Die Erfindung betrifft eine Vorrichtung zum Bestimmen der Rohdichte des Materials in einem Plattenstrang, umfassend eine Einrichtung zur Messung der Plattendicke des Materials.

Für eine Reihe von Produkten liegen Ausgangsmaterialien in einem Plattenstrang vor. Beispielsweise werden Faserplatten aus Faserschüttungen hergestellt, die ausgehend von diesen Schüttungen zu einem Plattenstrang verpresst werden und anschließend in einzelne Plattenstrangabschnitte unterteilt werden. Faserplatten können beispielsweise Holzfaserplatten sein.

Für eine hohe Qualität dieser Faserplatten ist es erforderlich, dass die Dichte des Materials über die gesamte Erstreckung der Platte bzw. über die gesamte Erstreckung eines Plattenabschnittes gleich ist. Damit ist verhindert, dass die Platte Einschlüsse von Materialien hoher oder niedriger Dichte enthält bzw. dass in der Platte Lufteinschlüsse vorhanden sind.

Zur Bestimmung der Rohdichte des Materials in einem Plattenstrang wird die Plattendicke zunächst erfasst. Darüber hinaus ist dann noch eine Bestimmung des Gewichtes beispielsweise eines Abschnittes des Plattenstranges notwendig, um die Rohdichte bestimmen zu können.

Im Stand der Technik ist für die Bestimmung des Gewichtes eine Waage eingesetzt worden. Diese Waage wird nach Vereinzelung der Plattenstrangabschnitte aus dem Plattenstrang im Produktionsprozess des Plattenstrangs eingesetzt, um die einzelnen Plattenstrangabschnitte nacheinander zu wiegen. Nachteilig ist, dass für das Wiegen eines Plattenstrangabschnittes eine bestimmte Zeitdauer benötigt wird. Dadurch wird die Fertigung von Plattenstrangabschnitten aufgehalten, da andere Bearbeitungsschritte der Plattenstrangabschnitte keine längeren Bearbeitungszeiten erfordern oder die erforderlichen Genauigkeiten nicht erreicht werden durch eine zu kurze Messzeit oder zu einem zu kleinen Nutzgewicht gegenüber dem Tara der Waage. Erwünscht wäre jedoch eine dauerhafte Genauigkeit von z.B. besser 1 % des Plattengewichtes. Dieses können die bekannten Waagen oft nicht liefern. Bekannt sind auch so genannte Flächengewichtswaagen, die mittels Radioisotopen oder Röntgengeräten streifenweise nach der Presse über Absorption der Strahlen die Flächenmasse bestimmen. Nachteilig an dieser Konstruktion ist jedoch, dass nicht über die gesamte Breite die Flächenmasse aufgenommen wird. Des Weiteren gibt es kaum Möglichkeiten der kurzfristigen Kalibrierung der Anlagen. Dadurch ist die dauerhafte Genauigkeit nicht gewährleistet.

Der Erfindung liegt die Aufgabe zugrunde, eine Vorrichtung der eingangs genannten Gattung aufzuzeigen, mit der die Rohdichte schneller bestimmbar ist.

Diese Aufgabe ist erfindungsgemäß dadurch gelöst, dass die Vorrichtung zumindest eine Röntgenquelle aufweist, dass die Röntgenquelle eine fächerförmige Strahlung aussendet und dass auf der in Bezug auf die Röntgenquelle anderen Seite des Plattenstranges zumindest ein Detektor angeordnet ist.

Bei der erfindungsgemäßen Vorrichtung wird auf den Einsatz einer Waage vollständig verzichtet. Die mit der Waage verbundenen längeren Messzeiten sind damit vorteilhaft nicht gegeben. Das Gewicht beispielsweise eines Plattenstranges wird nunmehr mithilfe einer Röntgenquelle und einem Detektor ermittelt. Die Röntgenquelle ist auf der einen Seite des Plattenstranges angeordnet, während der Detektor auf der anderen Seite des Plattenstranges angeordnet ist. Der Detektor nimmt damit von der Q:\IBSJAW\JAWDKA\2010003796.doc

Röntgenquelle ausgesandte Strahlung nach ihrem Durchtritt durch den Plattenstrang auf. Die Strahlung wird dabei quer zur Transportrichtung fächerförmig ausgestrahlt, so dass sich die Strahlung vorzugsweise über die gesamte Breite des Plattenstranges erstreckt, so dass bei gleichzeitigem Längstransport des Plattenstranges das Messgut vollständig erfasst ist.

Mit Hilfe der Röntgenquelle und des Detektors kann das Flächengewicht des Plattenstranges gemessen werden. Aus diesem Flächengewicht ist auf das Gewicht beispielsweise eines Plattenstranges rückschließbar und kann mit Hilfe der weiterhin wie bisher gemessenen Plattendicke die Rohdichte bestimmt werden.

Die Messung des Flächengewichtes erfolgt online in kürzester Zeit, so dass eine Verzögerung bei Herstellung von Plattenstrangabschnitten nicht gegeben ist.

Die Röntgenquelle sendet vorzugsweise eine niederenergetische Röntgenstrahlung aus. Diese Röntgenstrahlung wird vom Material des Plattenstranges absorbiert, aus der Stärke der Absorption ist das Flächengewicht bestimmbar. Für die Röntgenstrahlung kann ein Schutzgehäuse vorgesehen sein.

Die Röntgenquelle und der Detektor sind nach einer Weiterbildung der Erfindung an einem Ort in der Bearbeitungskette des Plattenstranges angeordnet, an dem aus dem Plattenstrang bereits voneinander vereinzelte Plattenstrangabschnitte ausgebildet sind. An diesem Ort ist im Stand der Technik auch die bekannte Waage angeordnet. Die einzelnen Plattenstrangabschnitte können unabhängig voneinander vermessen werden. Zwischen einzelnen Plattenstrangabschnitten ist ein Abstand ausgebildet, der bei der erfindungsgemäßen Vorrichtung vorzugsweise zur Kalibrierung genutzt werden kann. Das Kalibrieren kann ohne Anhalten der Gesamtvorrichtung zur Bearbeitung des Plattenstranges zwischen zwei Plattenstrangabschnitten erfolgen.

Nach einer nächsten Weiterbildung ist vorgesehen, dass aus dem aktuell verarbeiteten Plattenstrang für die Kalibrierung der Vorrichtung ein Musterstück herausgenommen ist. Musterstücke des Plattenstranges werden im angeschlossenen Labor ohnehin mehrmals täglich untersucht. Derartige Muster können dann auch für die Kalibrierung der erfindungsgemäßen Vorrichtung hergenommen werden.

Zur weiteren Ausbildung ist vorzugsweise vorgesehen, dass der Strahlenfächer der Röntgenquelle mehrere Messquadranten abdeckt. In den Strahlenfächer werden nach dieser Weiterbildung mehrere Messquadranten projiziert, mit denen der Strahlenfächer auf einzelne Abschnitte aufgeteilt werden kann. Diese einzelnen Abschnitte können dann bei der weiteren Betrachtung und Berechnung berücksichtigt werden. Die Seitenlängen der Messquadranten können etwa 4 cm bis 15 cm betragen.

Nach einer nächsten Weiterbildung der Erfindung kann noch vorgesehen sein, dass die Vorrichtung eine Messeinrichtung zum Messen der Feuchtigkeit im Material umfasst. Mit dieser kann die Feuchtigkeit im Plattenstrang gemessen werden und anschließend bei der Bestimmung der Rohdichte herausgerechnet werden. Damit kann die errechnete Rohdichte insbesondere bei Holzwerkstoffen auf einen absolut trockenen Wert gestellt werden.

Die Ergebnisse der Bestimmung der Rohdichte sind insbesondere aufgrund der Verwendung der Messquadranten gut darstellbar. Die Messwerte sind genau, so dass es geringe Standardabweichungen gibt.

Ein Ausführungsbeispiel der Erfindung, aus dem sich weitere erfinderische Merkmale ergeben, ist in der Zeichnung dargestellt. Es zeigen:
- Fig. 1:: eine erste Seitenansicht einer erfindungsgemäßen Vorrichtung zum Bestimmen der Rohdichte des Materials in einem Plattenstrang und
- Fig. 2:: eine weitere Seitenansicht der Vorrichtung gemäß Fig. 1.

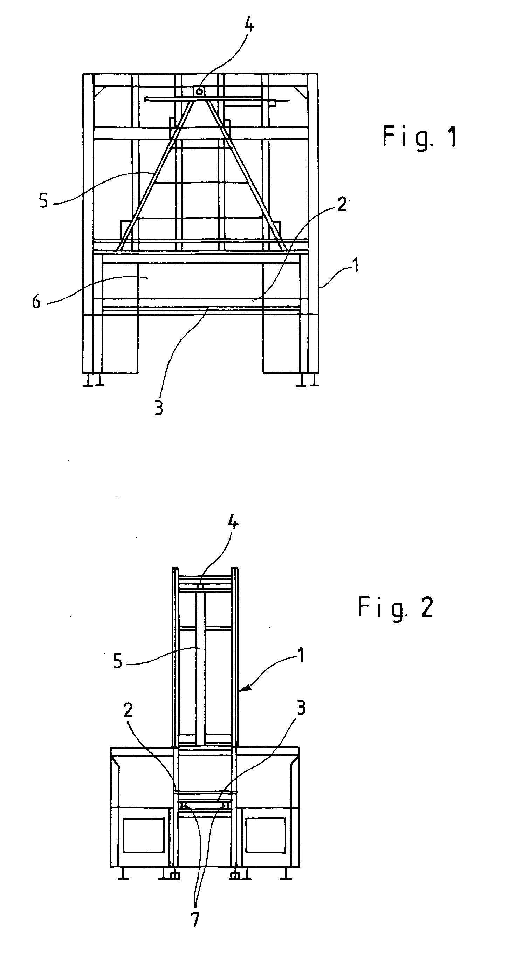

Die Vorrichtung in Fig. 1 weist ein Gestell 1 auf. Dieses Gestell 1 ist portalartig ausgebildet, es trägt eine Ebene 2 für einen Plattenstrang 6. Unterhalb dieser Ebene 2 ist im Gestell 1 ein Detektor 3 angeordnet. Dieser Detektor 3 ist zeilenartig ausgebildet.

Fig. 2 zeigt, dass der Detektor 3 schubladenartig auf Rädern 7 angeordnet ist.

Im oberen Bereich des Gestells 1 ist eine Röntgenquelle 4 gehalten. Diese Röntgenquelle 4 sendet eine fächerförmige Strahlung in Richtung der Ebene 2 aus. Die Strahlung ist in einem Gehäuse 5 aufgenommen. Die fächerförmige Strahlung der Röntgenquelle 4 erstreckt sich über die gesamte Breite des auf der Ebene 2 geführten Plattenstranges 6.

## Patentansprüche

1. Vorrichtung zum Bestimmen der Rohdichte des Materials in einem Plattenstrang, umfassend eine Einrichtung zur Messung der Plattendicke des Materials,
**dadurch gekennzeichnet,**
**dass** sie zumindest eine Röntgenquelle (4) aufweist, dass die Röntgenquelle (4) eine fächerförmige Strahlung aussendet und dass auf der in Bezug auf die Röntgenquelle (4) anderen Seite des Plattenstranges (6) zumindest ein Detektor (3) angeordnet ist.

2. Vorrichtung nach Anspruch 1, **dadurch gekennzeichnet, dass** die Röntgenquelle (4) eine niederenergetische Röntgenstrahlung aussendet.

3. Vorrichtung nach Anspruch 1 oder 2, dass die Röntgenquelle (4) und der Detektor (3) an einem Ort in der Bearbeitungskette des Plattenstranges (6) angeordnet sind, an dem aus dem Plattenstrang (6) bereits voneinander vereinzelte Plattenstrangabschnitte ausgebildet sind.

4. Vorrichtung nach einem der vorhergehenden Ansprüche, **dadurch gekennzeichnet, dass** sich die flächenförmige Strahlung über die gesamte Breite des Plattenstranges (6) erstreckt.

5. Vorrichtung nach einem der vorhergehenden Ansprüche, **dadurch gekennzeichnet, dass** aus dem aktuell verarbeiteten Plattenstrang (6) für ihre Kalibrierung ein Musterstück herausgenommen ist.

6. Vorrichtung nach einem der vorhergehenden Ansprüche, **dadurch gekennzeichnet, dass** der Strahlenfächer der Röntgenquelle (4) mehrere Messquadranten abdeckt.

7. Vorrichtung nach Anspruch 6, **dadurch gekennzeichnet, dass** die Seitenlängen der Messquadranten ca. 4 cm bis 20 cm betragen.

8. Vorrichtung nach einem der vorhergehenden Ansprüche, **dadurch gekennzeichnet, dass** sie eine Messeinrichtung zum Messen der Feuchtigkeit im Material umfasst.
